## Europäisches Patentamt
## European Patent Office
## Office européen des brevets

(11) Veröffentlichungsnummer : **0 065 481**
**B1**

(12) **EUROPÄISCHE PATENTSCHRIFT**

(45) Veröffentlichungstag der Patentschrift :
19.08.87

(51) Int. Cl.⁴ : **A 61 F   2/32**

(21) Anmeldenummer : **82730067.4**

(22) Anmeldetag : **18.05.82**

(54) **Hüftgelenkprothese mit gelochtem Hohlschaft.**

(30) Priorität : **18.05.81 DE 3120147**

(43) Veröffentlichungstag der Anmeldung :
**24.11.82 Patentblatt 82/47**

(45) Bekanntmachung des Hinweises auf die Patenterteilung : **19.08.87 Patentblatt 87/34**

(84) Benannte Vertragsstaaten :
**AT BE CH DE FR GB IT LI NL SE**

(56) Entgegenhaltungen :
DE-A- 2 049 111
DE-A- 2 617 749
DE-A- 2 851 598
DE-A- 2 933 237
DE-A- 2 941 265
**Flexural coupling of hip prothesis stem and femur: a simple method of theoretical analysis, Barberi et al. Engineering in Medicine, Band 7, Nr. 3, 1978**

(73) Patentinhaber : **MECRON MEDIZINISCHE PRODUKTE GMBH**
**Nunsdorfer Ring 25-27**
**D-1000 Berlin 48 (DE)**

(72) Erfinder : **Anapliotis, Emmanuel**
**Hauptstrasse 76**
**D-1000 Berlin 41 (DE)**
Erfinder : **Kranz, Curt, Dipl.-Ing.**
**Landshuter Strasse 5**
**D-1000 Berlin 30 (DE)**

(74) Vertreter : **Christiansen, Henning, Dipl.-Ing.**
**Dietrich-Schäfer-Weg 21**
**D-1000 Berlin 41 (DE)**

**Beschreibung**

Die Erfindung bezieht sich auf eine Hüftgelenk-Prothese der im Oberbegriff des Anspruchs 1 angegebenen Art.

Eine derartige Prothese ist bekannt aus der DE-A-28 51 598. Nachteilig bei dem bekannten Prothesenschaft ist, daß dieser in Bezug auf die umgebenden Knochenbereiche eine hohe Festigkeit aufweist, so daß der Schaft festigkeitsmäßig einen Fremdkörper bildet, der gegenüber dem Knochen Relativbewegungen ausführt, welche zu Knochenresorption führen können.

Es sind weiterhin gelochte Zusatzelemente für Schaftprothesen bekannt, welche als zusätzliche Trägerelemente zur Fixierung von Schaftprothesen bei Verwendung von Klebstoff oder Knochenzement dienen (DE-A-26 17 749). Bei dieser Prothese wird die Festigkeit der Gesamtanordnung aber im wesentlichen durch den Prothesenschaft und nicht durch das zusätzliche Trägerelement bestimmt. Im übrigen ist diese Konstruktion außerordentlich aufwendig.

Grundsätzlich werden die guten Anfangsergebnisse nach totalem Hüftgelenkersatz derzeit teilweise noch beeinträchtigt durch das bisher ungelöste Lockerungsproblem der zementierten Endoprothese. Die natürliche Krafteinleitung aus dem Hüftkopf über den Calcar in den Femurschaft wird durch den Einbau einer Endoprothese erheblich gestört. Der Einbau einer Prothese mit Kragen hat den Nachteil, daß örtlich am Prothesenaufsitz unphysiologische Druckkräfte auftreten. Die Prothese ohne Kragen führt zu unphysiologischen Umfangsspannungen im Calcar femoris und im coxalen Femurbereich aufgrund der Keilwirkung des konischen Prothesenschaftes. Beides, die örtlich hohen Druckkräfte wie auch die Umfangsspannungen, führen zu einer Resorption des Knochens im Bereich des Calcar. Infolge dieser Resorption kommt es auch bei der Prothese mit Kragen wegen der fehlenden Unterstützung durch den Calcar zu hohen Umfangsspannungen und nach einer gewissen Zeit zum Bruch im Zement. Die auf diese Art im oberen Bereich frei gewordene Prothese bewegt sich mit immer größeren Biegeverformungen im Zementköcher und es kommt zur totalen Auslockerung oder zum Bruch der Prothese.

Demgegenüber liegt der im Anspruch 1 charakterisierten Erfindung die Aufgabe zugrunde, eine Prothese anzugeben, bei der die Gefahr einer Lockerung verringert ist.

Die ermittelten Steifigkeiten in Längs- wie auch in Hauptbiegerichtung für unterschiedliche Materialien bei herkömmlichen Prothesentypen sind in der folgenden Tabelle in Relation zur Kortikalisröhre des Femurs gezeigt:

| Prothesenmaterial bzw. Prothesentyp | Längssteifigkeit Prothese/ Längssteifigkeit des Femur | Biegesteifigkeit Prothese/ Biegesteifigkeit des Femur |
|---|---|---|
| CoCr-Legierung | 2,11 | 1,79 |
| Stahl-Schmiedelegierung | 2,34 | 1,98 |
| Ti-Legierung | 1,22 | 1,03 |
| sog. Isoelastische Prothese | 0,99 | 0,84 |
| Hohlschaftprothese Ti-Legierung ohne Bohrung | 1,57 | 3,65 |
| Kortikalisröhre des Femur (Streuung) | 1,00 ($\pm$0,20) | 1,00 ($\pm$0,20) |

(Fortsetzung)

| Prothesenmaterial bzw. Prothesentyp | Längssteifigkeit Prothese/ Längssteifigkeit des Femur | Biegesteifigkeit Prothese/ Biegesteifigkeit des Femur |
|---|---|---|
| Durch Bohrungen angepaßte Hohlschaftsprothese Ti-Legierung (mögliche Variationsbreite) | 1,00 (±0,40) | 1,00 (±0,40) |

Aus der Tabelle ist ersichtlich, daß allein die Methode der Auswahl eines geeigneten Werkstoffs, wie z. B. Titan oder einem entsprechenden Kunststoff, — auch beispielsweise bei der sogenannten « Isoelastischen Prothese » (dargestellt bspw. in dem Aufsatz « Erste Erfahrungen mit einer zementlos isoelastischen Totalprothese der Hüfte » von Morscher, E. und Mathys, R. im Zorthop, 113, (1975), Seiten 745 bis 749) nicht ausreichend ist, um sowohl die Biege- als auch die Längssteifigkeit der Prothese der Kortikalisröhre anzupassen.

Eine Verringerung der Biegesteifigkeit durch das große Flächenträgheitsmoment der erfindungsgemäßen Hohlschaftprothese wird durch ein geeignetes Anbringen von Schwächungen in Form von lochförmigen Aussparungen bzw. gegebenenfalls durchgehenden Bohrungen unter gleichzeitiger Beachtung der resultierenden Längssteifigkeit erzielt. Es zeigt sich, daß der Querschnitt an der Innen- wie an der Außenseite des Schaftes durch große Bohrungen mit etwa 5 bis 8 mm Durchmesser (zur Vermeidung von Kerbwirkungen) so weit geschwächt werden kann, daß die resultierende Biegesteifigkeit der in entsprechenden Höhenschichten der Kortikalisröhre vorhandenen Biegesteifigkeit entspricht.

Durch weitere Bohrungen kleineren Durchmessers, die im hinteren und/oder vorderen Bereich des Prothesenschafts vorgesehen sind, wird die Möglichkeit des Einwachsens von Knochensubstanz verbessert und die Biegesteifigkeit gegebenenfalls zusätzlich herabgesetzt, die Längssteifigkeit aber nur umwesentlich verringert.

Einer Weiterbildung der Erfindung liegt die Erkenntnis zugrunde, daß diese zusätzlichen Maßnahmen geeignet sind, die erfindungsgemäße Prothese insbesondere getrennt bezuglich der Längs- und der Biegesteifigkeit derjenigen der Kortikalisröhre des Knochens anzupassen und damit bei zementfreier Befestigung die Voraussetzungen für eine lange Tragedauer ohne Gefahr einer Prothesenlockerung schaffen.

Die erfindungsgemäße Prothese weist eine grössere Nachgiebigkeit auf als eine solche mit Vollschaft, wobei die Nachgiebigkeit durch Wahl der Dicke des Materials (im Bereich vor bis 3 mm) den jeweiligen Gegebenheiten angepaßt werden kann. Die Locher — in Form von Aussparungen oder (durchgehenden) Bohrungen — Hohlkörper der Prothese ermöglichen das Einwachsen von Knochenmaterial, wodurch der erfindungsgemäße Prothesenschaft zusätzlichen Halt bekommt. Das Auffüllen des Hohlkörpers mit Knochengranulat beschleunigt das Einwachsen der Prothese.

Weitere Einzelheiten der Erfindung ergeben sich aus der Zeichnung. Darin zeigen :

Figur 1  einen Längsschnitt durch eine erste Ausführungsform der Erfindung ;
Figur 2  einen Schnitt gemäß der Linie II-II der Figur 1 ;
Figur 3  einen Schnitt nach der Linie III-III der Figur 2 ;
Figur 4  einen Längschnitt durch eine zweite Ausführungsform der Erfindung ;
Figur 5  einen Schnitt nach der Linie V-V der Figur 4 ;
Figur 6  einen Schnitt nach der Linie VI-VI der Figuren 5 und
Figur 7  eine weitere Ausführungsform eines Prothesenschafts gemäß der Erfindung.

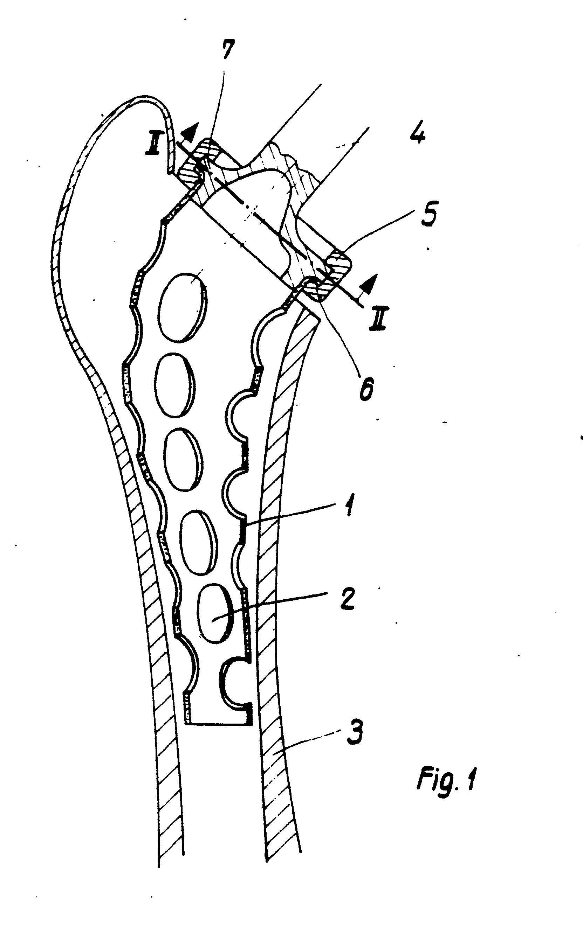

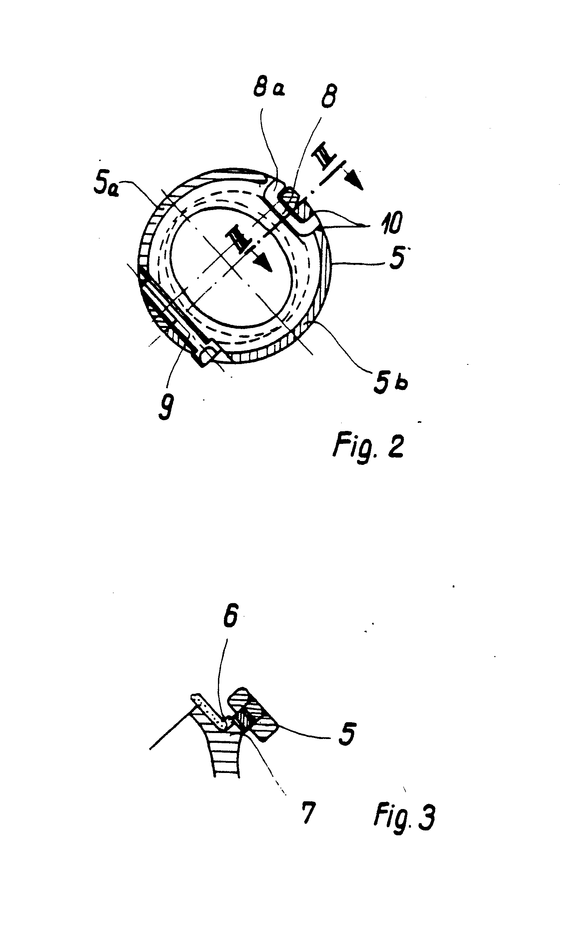

In den Figuren 1 bis 3 ist ein gekrümmter Hohlschaft 1 einer Hüftgelenk-Prothese dargestellt, der mit durchgehenden Löchern 2 versehen ist. Neben der in der Zeichenebene verlaufenden Krümmung ist eine weitere, geringere Krümmung des Schaftes in einer senkrecht dazu gerichteteten Ebene vorgesehen, welche zur Anpassung an den linken bzw. rechten Femur dient.

Die Prothese ist in einen Oberschenkelknochen 3 eingesetzt und weist einen Hals 4 zum Aufsetzen einer — nicht dargestellten — Gelenkkugel auf. Ein Ring 5 besteht aus zwei Hälften 5a und 5b und umgreift einen an dem Schaft 1 angeordneten Ansatz 6 und einen am Hals 4 angebrachten, weiteren Ansatz 7. Ein Haken 8 ist in einem Bereich 10 angeschweißt. Sein Schenkel 8a sitzt locker in einer Ausnehmung der Ringhälfte 5a. Nach Lösen einer an der dem Haken gegenüberliegenden Seite angebrachten Schraube 9 kann die Hälfte 5a von der Hälfte 5b weggeklappt werden, was zur Folge hat, daß der Hals 4 und damit die nicht dargestellte Gelenkkugel vom Schaft 1 zwecks Austauschs der Gelenkkugel auch ohne Schaft entfernbar ist. Der Haken 8 bildet zugleich eine Sicherung gegen Verdrehung des Gelenkkopfes gegenüber dem Schaft 1.

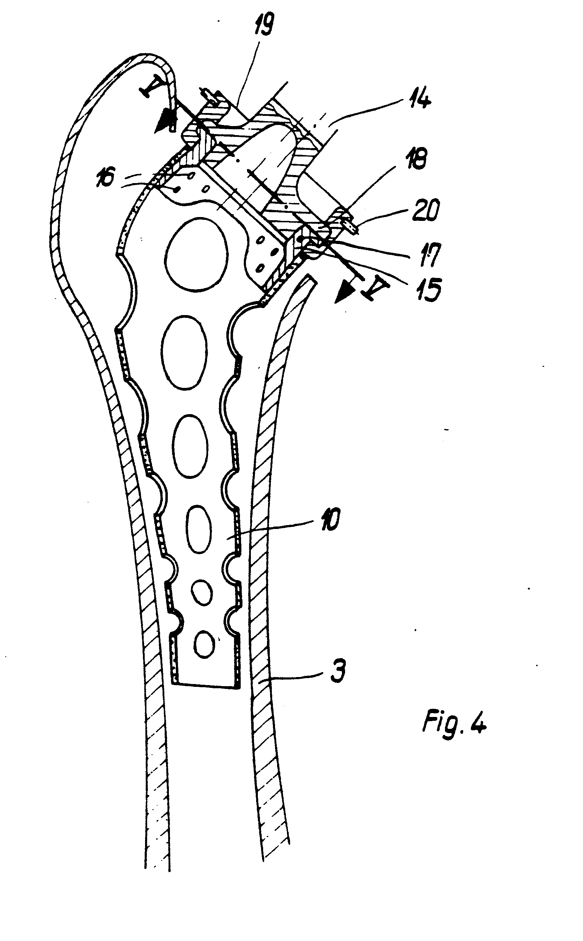

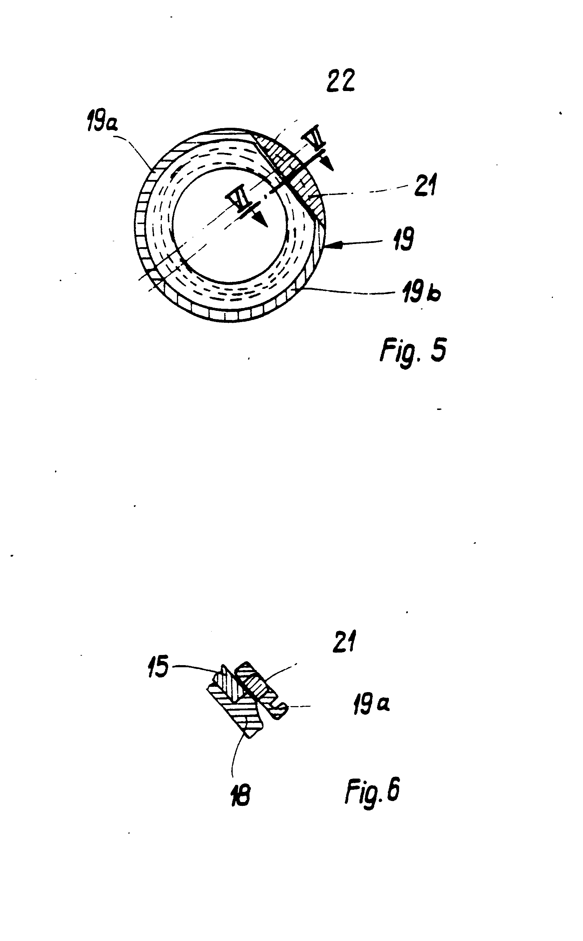

Die Ausführungsform der Figuren 4 bis 6 besteht ebenfalls aus einem Schaft 10, einer nicht dargestellten Gelenkkugel mit einem Hals 14. In diesem Falle ist ein Einsatz 15 am oberen Ende des Schaftes 10 vorgesehen. Der Einsatz 15 und der Schaft 10 sind in Punkten 16 miteinander verschweißt. Der Einsatz 15 besitzt einen Ansatz 17, der an einem Ansatz 18 des Halses 14 anliegt. Beide Ansätze werden von einem aus zwei Teilen 19a und 19b bestehenden Ringteil 19 umfaßt. Beide Hälften des Ringteils 19 werden von einem Spiralring 20 zusammengehalten. Die Verdrehungsicherung wird in diesem Falle von einem Segment 21 gebildet, das bei 22 an der einen Ringteil 19a festgeschweißt ist und den entsprechenden abgeflachten Kragen 17 und 18 anliegt. Der Schaft besteht aus dem Blech einer Titanlegierung oder einem Faser-Verbund-Werkstoff, wie beispielsweise einem mit Kohlenstoffasern verstärktem Kunststoff.

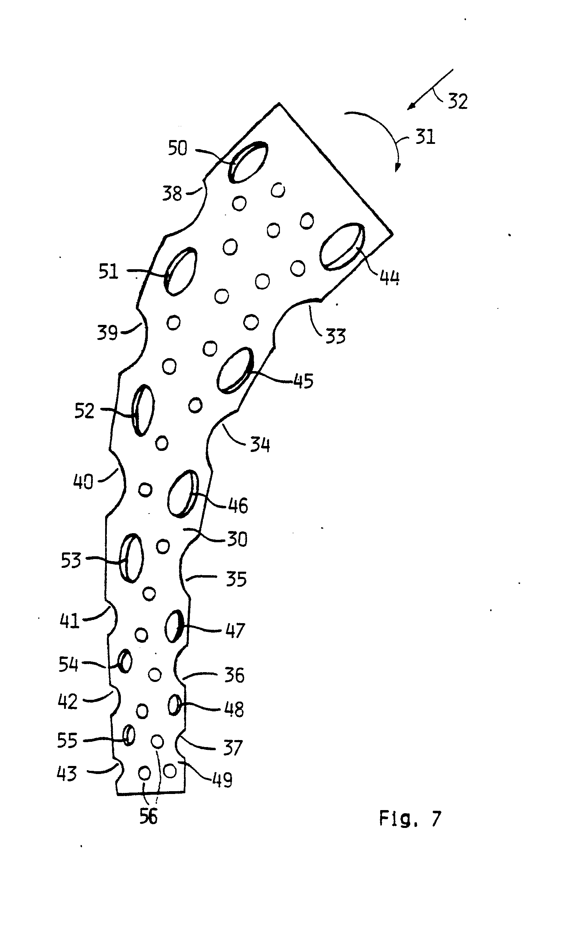

In Figur 7 ist ein Prothesenschaft 30 dargestellt, wie er entsprechend den vorteilhaften Weiterbildungen der Erfindung optimal an die örtlichen Gegebenheiten der Knochenfestigkeit im Femurbereich anpaßbar ist. Während die Biegebelastung der Prothese beim Tragen vorwiegend durch (in der Zeichnung) von oben her wirkende Kräfte bestimmt wird, welche eine Biegemoment (Pfeil 31) mit der Tendenz zur Folge haben, die dargestellte ursprünglich vorhandene Krümmung zu verstärken. Die Längssteifigkeit ist im Hinblick auf Druckkräfte festzulegen, welche in Richtung der Mittelachse der Prothese wirken. Diese Belastung erfolgt ebenfalls von oben her, die Kräfte werden jedoch senkrecht zur Oberkante des Schaftbereichs eingeleitet (Pfeil 32).

Die Überleitung der am oberen Anschluß der Gelenkkugel auftretenden Kräfte in den Femur erfolgt kontinuierlich, wobei die Verjüngung des Schaftes (sowie gegebenenfalls der Materialstärke des Schaftmaterials) und die damit verbundene Stabilitätsverminderung der Verringerung der insgesamt zu übertragenen Kräfte entspricht. Die Bemessung der Festigkeit, d. h. die Schwächung durch gezielt vorzusehende lochförmige Aussparungen größeren Durchmessers erfolgt dabei schichtweise, wobei die Aussparungen bevorzugt im Bereich des Innen- bzw. Außenumfangs der Hauptkrümmung angeordnet sind, wie sie der Form bekannter Femurprothesen entspricht.

Dabei sind Löcher größeren Durchmessers 33 bis 43 direkt auf der inneren bzw. äußeren Linie der Ebene maximaler Krümmung, welche in etwa parallel zur Mittelachse der Prothese verlaufen, angeordnet. Diese Löcher sind im Querschnitt analog zum Durchmesser des zugehörigen Schaftquerschnitts zum der Gelenkkugel gegenüberliegenden Ende hin verkleinert. Den Aussparungen 33 bis 43 benachbart sind weitere lochförmige Aussparungen 44 bis 55, die neben den Schnittlinien des Prothesenmantels mit der Ebene größter Krümmung (Zeichenebene Figur 7) angeordnet sind und die Löcher 33 bis 43 in ihrer Funktion ergänzen. Der Durchmesser ist jeweils geringfügig geringer als derjenige der erstgenannten Löcher. Die lochförmigen Aussparungen sind bevorzugt kreisrund ausgebildet, um Kerbwirkungen zu vermeiden.

Weitere in den übrigen Bereichen des Prothesenschaftes 30 verteilte und versetzt angeordnete Löcher 56 kleineren Durchmessers gehen nur geringfügig in die Festigkeitsberechnungen zur Ermittlung der Längssteifigkeit der Prothese ein und begünstigen das Einwachsen von Knochensubstanz zur Verfestigung des Prothesensitzes. Sie sind bei entsprechender Bemessung jedoch geeignet, die Biegesteifigkeit der Prothese soweit herabzusetzen, daß sie dem entsprechenden Femurbereich angepaßt ist, wenn die Längssteifigkeit bereits durch die Bemessung der größeren Aussparungen festgelegt ist. Auf diese Weise ist auch bei Wahl unterschiedlicher Materialien die Anwendung der erfindungsgemäßen Lehre möglich.

Die in Figur 7 dargestellte Krümmung entspricht in ihrer Form dem Verlauf der Hauptkraftlinien im Prothesenschaft.

Um eine noch verbesserte Anpassung an die unterschiedlichen Gegebenheiten im linken und rechten Femur zu erreichen, ist eine zusätzliche Krümmung um eine Achse vorgesehen, welche horizontal unter bzw. oberhalb der Zeichenebene, d. h. der Hauptkrümmungsebene, erfolgt. Diese Krümmung ist wesentlich geringer als die aus der Zeichnung ersichtliche Hauptkrümmung und wirkt sich bevorzugt auf den oberen Teil des Schaftes aus.

**Patentansprüche**

1. Hüftgelenkprothese mit einem Hohlschaft (1, 10, 30), der durch lochförmige Aussparungen (2, 33 bis 55) örtliche Materialschwächungen aufweist, dadurch gekennzeichnet, daß die lochförmigen Aussparungen derart angeordnet und ausgebildet sind, daß der Hohlschaft bezüglich seiner Längs- und/oder Biegesteifigkeit an die Längs- bzw. Biegesteifigkeit des umgebenden Knochenbereichs angepaßt ist, und daß der Schaft (1, 10, 30) im übrigen eine im wesentlichen konstante Materialstärke im Bereich von 1 bis 3 mm aufweist.

2. Hüftgelenkprothese nach Anspruch 1, dadurch gekennzeichnet, daß die lochförmigen Aussparungen rund mit Durchmessern im Bereich von 5 bis 8 mm ausgebildet sind.

3. Hüftgelenkprothese nach Anspruch 2, dadurch gekennzeichnet, daß neben den runden lochförmigen Aussparungen (33 bis 55) mit Durchmessern im Bereich von 5 bis 8 mm weitere gleichmäßig verteilte lochförmige Aussparungen (66) kleineren Durchmessers vorgesehen sind.

4. Hüftgelenkprothese nach einem der Ansprüche 2 oder 3, dadurch gekennzeichnet, daß die runden lochförmigen Aussparungen (33 bis 55) im Bereich des Innen- und/oder des Außenbogens vorgesehen sind.

5. Hüftgelenkprothese nach einem der vorangehenden Ansprüche, dadurch gekennzeichnet, daß jeweils ein einen Abschnitt der Längsachse des Schaftes umgebendes Segment bezüglich seiner elastischen und/oder plastischen Verformungseigenschaften separat dem umgebenden Knochenbereich angepaßt ist.

6. Hüftgelenkprothese nach einem der vorangehenden Ansprüche, dadurch gekennzeichnet, daß die Gelenkkugel, vorzugsweise aus Keramik bestehend, abnehmbar ist.

7. Hüftgelenkprothese nach Anspruch 6, dadurch gekennzeichnet, daß an dem freien Ende des Kugelhalses (4, 14) ein Ansatz (7, 18) angeformt ist, daß das dem Hals angrenzende Ende des Schaftes (1, 10) ebenfalls einen Ansatz (6, 17) aufweist und daß beide Ansätze von einem entfernbaren, vorzugsweise aus zwei Hälften bestehenden Haltering (5, 19) umfaßt werden.

8. Hüftgelenkprothese nach einem der vorangehenden Ansprüche, dadurch gekennzeichnet, daß das Schaftmaterial zu dem der Gelenkkugel abgewandten Ende des Schaftes hin in seiner Dicke verringert ist.

9. Hüftgelenkprothese nach einem der vorangehenden Ansprüche, dadurch gekennzeichnet, daß eine zusätzliche geringere Krümmung des Prothesenschafts um eine Achse vorgesehen ist, die parallel zu der Ebene in der die stärkere Krümmung verläuft, und senkrecht zur Prothesenlängsachse gerichtet ist.


## Claims

1. An artificial hip-joint having a hollow shank (1, 10, 30) which has local weakening of the material as a result of openings (2, 33 to 55) in the form of holes, characterised in that the openings in the form of holes are arranged and formed in such a manner that the hollow shank is adapted, with regard to its longitudinal rigidity and/or bending resistance, to the longitudinal rigidity or bending resistance of the surrounding bone region, and that otherwise the shank (1, 10, 30) has a substantially constant material thickness in the range from 1 to 3 mm.

2. An artificial hip-joint as claimed in Claim 1, characterised in that the openings in the form of holes are constructed round with diameters in the range from 5 to 8 mm.

3. An artificial hip-joint as claimed in Claim 2, characterised in that apart from the round openings (33 to 55) in the form of holes with diameters in the range from 5 to 8 mm, further openings (66) in the form of holes of smaller diameter are provided, uniformly distributed.

4. An artificial hip-joint as claimed in either of Claims 2 or 3, characterised in that the round openings (33 to 55) in the form of holes are provided in the region of the inner and/or outer curve.

5. An artificial hip-joint as claimed in any preceding Claim, characterised in that one segment surrounding a portion of the longitudinal axis of the shank is adapted separately, in each case, with regard to its elastic and/or plastic deformation properties, to the surrounding bone region.

6. An artificial hip-joint as claimed in any preceding Claim, characterised in that the spherical part of the joint, which preferably consists of ceramic, is removable.

7. An artificial hip-joint as claimed in Claim 6, characterised in that a projection (7, 18) is formed on the free end of the neck (4, 14) of the spherical part, that the end of the shank (1, 10) adjacent to the neck likewise comprises a projection (6, 17) and that the two projections are encircled by a removable retaining ring (5, 19) preferably consisting of two halves.

8. An artificial hip-joint as claimed in any preceding Claim, characterised in that the material of the sank is reduced in thickness towards the end of the shank remote from the spherical part of the joint.

9. An artificial hip-joint as claimed in any preceding Claim, characterised in that an additional relatively slight curvature of the shank of the artificial joint is provided about an axis which extends parallel to the plane in which the greater curvature extends and is directed perpendicular to the longitudinal axis of the artificial joint.


## Revendications

1. Prothèse d'articulation de hanche comprenant une tige creuse (1, 10, 30) qui présente des affaiblissements locaux de la matière, réalisés par des évidements en forme de trous (2, 33 à 55), caractérisée en ce que les évidements en forme de trous sont disposés et configurés de telle manière que la tige creuse soit adaptée, sous l'aspect de sa rigidité longitudinale et/ou de sa rigidité en flexion, à la rigidité longitudinale et/ou à la rigidité en flexion de la région osseuse qui l'entoure et en ce que la tige (1, 10, 30) présente par ailleurs une épaisseur de matière sensiblement constante, dans l'intervalle de 1 à 3 mm.

2. Prothèse d'articulation de hanche selon la revendication 1, caractérisée en ce que les évidements en forme de trous sont d'une forme ronde, avec des diamètres dans l'intervalle de 5 à 8 mm.

3. Prothèse d'articulation de hanche selon la revendication 2, caractérisée en ce que, en supplément des évidements ronds en forme de trous (33 à 55) possédant des diamètres dans l'intervalle de 5 à 8 mm, il est prévu d'autres évidements en forme de trous (66) de plus petit diamètre et uniformément répartis.

4. Prothèse d'articulation de hanche selon l'une des revendications 2 et 3, caractérisée en ce que les évidements ronds en forme de trous (33 à 55) sont prévus dans la région de l'arc intérieur et/ou de l'arc extérieur.

5. Prothèse d'articulation de hanche selon l'une des revendications précédentes, caractérisée en ce que chaque segment qui entoure une partie donnée de l'axe longitudinal de la tige est adapté individuellement à la région osseuse qui l'entoure sous l'aspect de ses propriétés de déformation élastique et/ou plastique.

6. Prothèse d'articulation de hanche selon l'une des revendications précédentes, caractérisée en ce que la tête d'articulation, de préférence réalisée en céramique, est amovible.

7. Prothèse d'articulation de hanche selon la revendication 6, caractérisée en ce qu'à l'extrémité libre du col du fémur (4, 14), est formée une collerette (7, 18), en ce que l'extrémité de la tige (1, 10) qui est adjacente au col, présente également une collerette (6, 17) et en ce que les deux collerettes sont entourées d'une bague de retenue (5, 19), qui est faite de préférence de deux moitiés.

8. Prothèse d'articulation de hanche selon l'une des revendications précédentes, caractérisée en ce que la matière de la tige est d'une épaisseur décroissante en direction de l'extrémité de la tige qui est à l'opposé de la tête d'articulation.

9. Prothèse d'articulation de hanche selon l'une des revendications précédentes, caractérisée en ce qu'elle présente une légère courbure additionnelle de la tige de prothèse, autour d'un axe qui s'étend parallèlement au plan dans lequel s'étend la plus forte courbure, et est orienté perpendiculairement à l'axe longitudinal de la prothèse.

Fig. 1

Fig. 2

Fig. 3

Fig. 4

Fig. 5

Fig. 6

Fig. 7